# EUROPÄISCHE PATENTSCHRIFT

(11) **EP 0 871 609 B1**
(45) Veröffentlichungstag und Bekanntmachung des Hinweises auf die Patenterteilung: **07.11.2001**
(21) Anmeldenummer: 96939936.9
(22) Anmeldetag: 28.11.1996
(51) Int. Cl.: C07C 251/42, C07C 239/20, C07C 323/47, A01N 43/14, A01N 43/10

(54) **CYCLOHEXENONOXIMETHER-METALLSALZE**
CYCLOHEXENONE OXIME ETHER METAL SALTS
SELS METALLIQUES D'ETHER D'OXIME DE CYCLOHEXENONE

(30) Priorität: 05.12.1995 DE 19545212
(43) Veröffentlichungstag der Anmeldung: 21.10.1998
(73) Patentinhaber: BASF AKTIENGESELLSCHAFT, 67056 Ludwigshafen (DE)
(72) Erfinder: BRATZ, Matthias, D-67117 Limburgerhof (DE); JÄGER, Karl-Friedrich, D-67117 Limburgerhof (DE); BENOIT, Remy, F-64300 Lanneploa (DE); RANG, Harald, D-67122 Altrip (DE); MISSLITZ, Ulf, D-67433 Neustadt (DE); WESTPHALEN, Karl-Otto, D-67346 Speyer (DE)
(86) Internationale Anmeldenummer: EP9605255
(87) Internationale Veröffentlichungsnummer: WO9720807

(56) Entgegenhaltungen:
- EP-A- 0 368 227
- WO-A-96/29869
- DE-A- 3 941 160
- DE-A- 4 014 983
- DE-A- 4 014 986
- DE-A- 4 014 988
- US-A- 4 741 768
- US-A- 5 228 896
- DATABASE WPI Week 8722 Derwent Publications Ltd., London, GB; AN 87-153209 XP002025923 in der Anmeldung erwähnt & JP 62 089 653 A (NIPPON SODA KK)

## Beschreibung

Die vorliegende Erfindung betrifft neue Cyclohexenonoximether-Metallsalze der Formel I 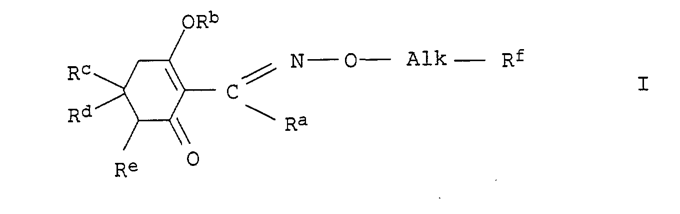 in der die Variablen folgende Bedeutungen haben:
- R^{a}: C₁-C₆-Alkyl;
- R^{b}: ein Lithiumion;
- R^{c}: einen 6- oder 7-gliedrigen gesättigten oder ein- oder zweifach ungesättigten Heterocyclus, der neben Kohlenstoffatomen ein oder zwei Sauerstoff- oder Schwefelatome oder ein Sauerstoff- und ein Schwefelatom als Ringglieder enthält, und der gewünschtenfalls noch einen bis drei Substituenten tragen kann, jeweils ausgewählt aus der Gruppe bestehend aus Hydroxyl, Halogen, C₁-C₄-Alkyl, C₁-C₄-Halogenalkyl, C₁-C₄-Alkoxy und C₁-C₄-Alkylthio;
- R^{d}: Wasserstoff;
- R^{e}: Wasserstoff;
- Alk: eine 3- bis 6-gliedrige Alkylenkette, die gewünschtenfalls einen C₁-C₃-Alkylsubstituenten tragen kann und die neben Methylen- oder Methineinheiten ein Sauerstoff- oder Schwefelatom als Brückenglied enthält;
- R^{f}: Halogenphenyl.

Außerdem betrifft die Erfindung
- die Verwendung der Verbindungen I als Herbizide,
- herbizide Mittel, welche die Verbindungen I als wirksame Substanzen enthalten,
- Verfahren zur Herstellung der Verbindungen I und von herbiziden Mitteln unter Verwendung der Verbindungen I, sowie
- Verfahren zur Bekämpfung von unerwünschtem Pflanzenwuchs mit den Verbindungen I.

Bei Pflanzenschutzmitteln ist es grundsätzlich wünschenswert, die spezifische (hier: herbizide) Wirkung und die Wirkungssicherheit zu verbessern. Wesentlich ist dabei die chemische Stabilität des Wirkstoffs und die Lagerstabilität der fertigen Pflanzenschutzmittelformulierung. Darüberhinaus ist auch eine gewisse Stabilität des Wirkstoffes selbst in der Umwelt, für eine kurze Zeit nach der Ausbringung, wünschenswert.

Aus der EP-A 266 068 ist bereits bekannt, daß Herbizide aus der Stoffklasse der Cyclohexenonoximether eine Tendenz zur Zersetzung aufweisen. Besonders kritisch ist dabei die Lagerung ihrer anwendungsfertigen Aufbereitungen über einen längeren Zeitraum bei erhöhten Temperaturen.

Neben dieser Langzeitstabilität ist ferner die kurzzeitige thermische Belastbarkeit einer Verbindung bei deren Herstellung und Aufbereitung als Pflanzenschutzmittel, z.B. während Reinigungsoder Trocknungsoperationen, von entscheidender Bedeutung.

In der Substanzklasse der Cyclohexenonoximether werden üblicherweise neben den freien Verbindungen auch deren Salze als herbizid wirksam gelehrt (siehe z.B. die JP-A 59/163363: Natrium-, Kalium-, Calcium-, Magnesium-, Barium-, Nickel-, Mangan-, Kobalt-, Zink- und Eisensalze).

Gegenstand der JP-A 78/034753 ist die Herstellung von Natrium-, Kalium-, Calcium-, Magnesium-, Barium-, Nickel-, Kupfer-, Mangan-, Kobalt-, Zink-, Eisen- und Silbersalzen bestimmter 2-Alkyloxy-, 2-Alkenyloxy-, 2-Alkinyloxy- und 2-Benzyloxyiminoalkyl-cyclohexenone.

Aus DE-A 39 41 160 und JP-A 62/089653 ist zwar eine erhöhte Stabilität strukturell ähnlicher Lithiumsalze bekannt, über die Hygroskopizität derartiger Salze wird jedoch keine Aussage gemacht.

In der U.S. 4,741,768 werden die Kupfer-, Lithium- und Magnesiumsalze von bestimmten 2-[1-(3-Chlorallyloxyimino)-alkyliden]-cyclohexan-1,3-dionen mit verbesserter Lagerstabilität im Hinblick auf Temperatur und (Luft)feuchtigkeit gelehrt. Allerdings wird in Spalte 10, Tabelle A ein spezielles Lithiumsalz als stark hygroskopisch bezeichnet.

In der EP-A 085 530 und der U.S. 4,952,722 sind neben vielen Cyclohexenonoximethern auch einige Salze aufgelistet, wobei das Kation in der EP-Schrift Lithium, Natrium, Kalium oder ½ Kupfer und in der U.S.-Schrift Lithium, Natrium, ½ Kupfer oder ½ Nickel ist. Den Salzen wird jedoch kein spezieller Vorteil zugeschrieben.

In der DE-A 39 41 160 werden lagerstabile Salze von Acyl-cyclohexandion-oximethern mit herbizider und das Pflanzenwachstum regulierender Wirkung gelehrt, die auch eine gegenüber den freien Verbindungen verbesserte Bodenstabilität aufweisen sollen. Explizit genannt werden Salze mit einem Lithium-, Natrium-, Kalium-, ½ Magnesium-, ½ Calcium-, ½ Barium, ½ Kupfer(II)- oder ½ Zink-Kation. Es wird aber auch darauf hingewiesen, daß die Salze bei der Herstellung oft mit Lösungsmittel zusammen auskristallisieren. Dies wäre aber für eine spätere Verwendung als Pflanzenschutzmittel nachteilig.

Der Wirkstoff 2-(1-Allyloxyiminobutyl)-4-methoxycarbonyl-5,5-dimethyl-3-oxo-cyclohexenol (Common name: ALLOXYDIM) schließlich wird als Natrium-Salz in den Handel gebracht. (vgl. Meded.FAc. Landbouwwet. 1977, 42(2,Pt.2), 1597-1614; The Pesticide Manual, 9th Ed. 1991, S. 21).

Da also bisher die Salze von Cyclohexenonoximethern mit den verschiedensten Kationen beschrieben werden, läßt sich für die I zugrundeliegenden freien Verbindungen (II; R^{b} wäre also H) nicht ableiten, welche Salze besonders gute Eigenschaften im Hinblick auf Lager- und Temperaturstabilität, Anfälligkeit gegen Feuchtigkeit oder biologischer Wirkung haben.

Aufgabe der vorliegenden Erfindung war es nun, ausgehend von den Cyclohexenonoximethern (II); herbizide Wirkstoffe mit besseren physikalischen Eigenschaften zur Verfügung zu stellen.

Demgemäß wurden die vorliegenden Cyclohexenonoximether-Metallsalze der Formel I gefunden. Ferner wurden herbizide Mittel gefunden, die die Verbindungen I enthalten und eine sehr gute herbizide Wirkung besitzen. Weiterhin wurde gefunden, daß die Salze I herausragende Lagerstabilität haben. Daneben sind diese Salze nicht hygroskopisch und thermisch belastbarer als die freien Säuren (II), was in DSC-Unternehmungen durch eine beginnende thermische Zersetzung erst oberhalb von 100°C zum Ausdruck kommt.

Außerdem wurden Verfahren zur Herstellung der Verbindungen I, von sie enthaltenden Mitteln und Verfahren zur Bekämpfung von unerwünschtem Pflanzenwuchs mit den Verbindungen I gefunden.

Die den Salzen I zugrundeliegenden freien Cyclohexenonoximether der Formel II 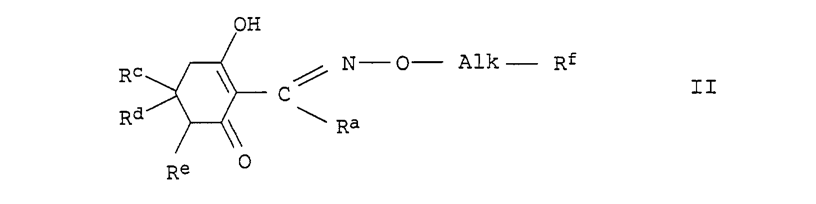 wobei R^{a}, R^{c} bis R^{f} und Alk die gleichen Bedeutungen wie bei Formel I haben, sind z.B. aus der DE-A 40 14 986 bekannt.

Als Wirkstoffe kommen sowohl die reinen Enantiomeren I als auch deren Racemate oder Diastereomerengemische in Betracht.

Die für R^{a} und R^{c} bis R¹ sowie für die Substituenten an "Alk" oder an (Hetero)cyclen genannten organischen Molekülteile stellen Sammelbegriffe für individuelle Aufzählungen der einzelnen Gruppenmitglieder dar. Sämtliche Kohlenstoffketten, also alle Alkyl-, Halogenalkyl-, Alkoxy- und Alkylthio-Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Brom, Chlor oder Iod, insbesondere für Fluor oder Chlor.

Besonders bevorzugt sind diejenigen Cyclohexenonoximether-Metallsalze der Formel I, bei denen die Substituenten die folgenden Bedeutungen haben, und zwar jeweils für sich allein oder in Kombination:
- R^{a}: Ethyl- oder n-Propyl;
- R^{c}: Tetrahydropyran-3-yl, Tetrahydropyran-4-yl oder Tetrahydrothiopyran-3-yl;
- Alk: Ethylenoxy oder 1,2-Propylenoxy;
- R^{f}: 4-Fluorphenyl oder 4-Chlorphenyl.

Als besonders geeignet haben sich die in Tabelle 1 aufgeführten Cyclohexenonoximether-Metallsalze erwiesen:

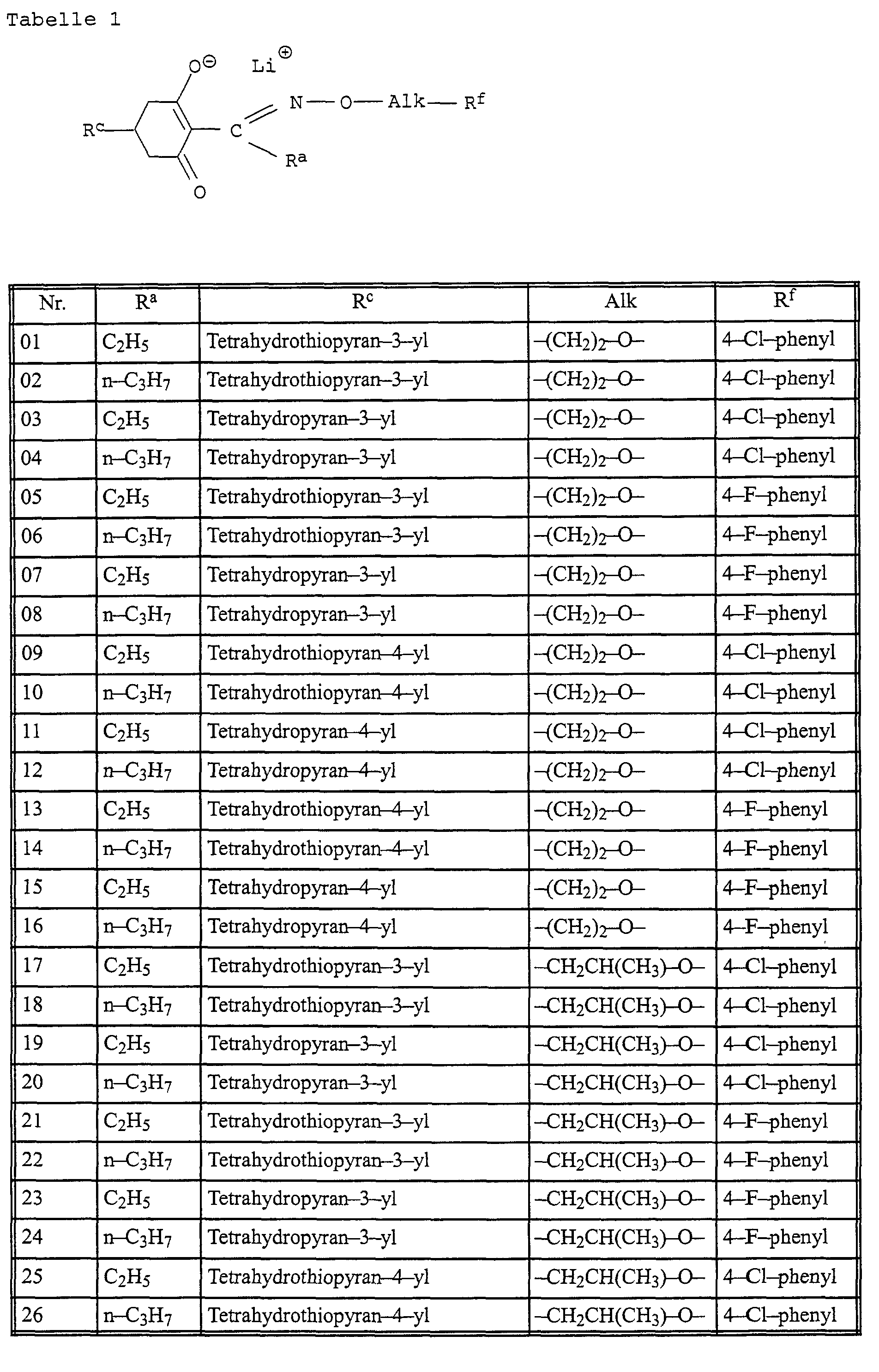

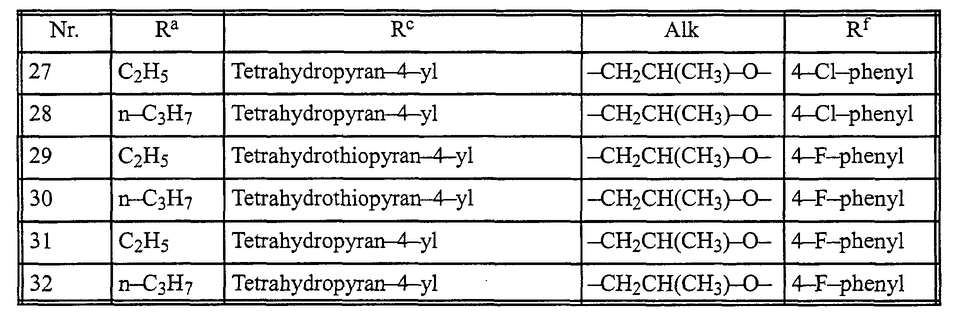

Nach den bisherigen Erkenntnissen hat sich die Verbindung Nr. 18 als ganz besonders vorteilhaft erwiesen.

Im allgemeinen sind die Cyclohexenonoximether-Metallsalze I durch Umsetzung der entsprechenden freien Verbindung (II) mit einem Hydrid, Hydroxid, Alkoholat oder Carbonat des gewünschten Metall-Ions zugänglich.

Besonders empfehlenswert ist es dabei, von einer Lösung von (II) auszugehen, wie sie bei dessen Herstellung aus III und IV (siehe z.B. EP-A 456 112) anfällt, also ohne vorherige Isolierung von II:

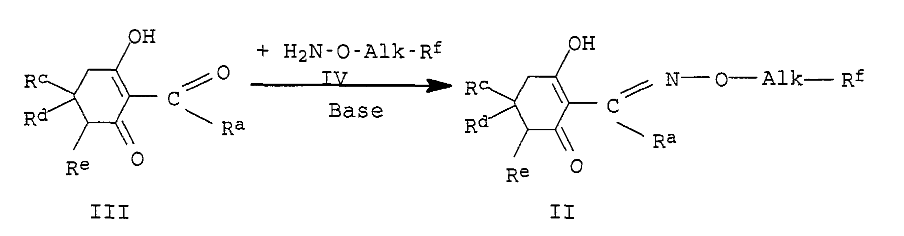

Geeignete Lösungsmittel für II sind insbesondere Wasser oder mit Wasser mischbare Lösungsmittel, z.B. Ether wie Tetrahydrofuran und Dioxan oder niedere Alkohole wie Methanol und Ethanol.

Das Metallhydrid, -hydroxid, -alkoxid oder Metallcarbonat wird in der Regel gelöst oder suspendiert in einem der o.g. Solventien oder in fester Form eingesetzt.

Die Neutralisation von II wird normalerweise bei einer Temperatur von (-100)°C bis 200°C, bevorzugt von (-40)°C bis 120°C, insbesondere von (-10)°C bis 80°C, vorgenommen.

Das gebildete Reaktionswasser wird dabei vorzugsweise mittels azeotroper Destillation entfernt. Nach Abtrennen der niedrigsiedenden Anteile erhält man dann das trockene Cyclohexenonoximether-Metallsalz I in kristalliner oder amorpher Form.

Eine Verfahrensvariante besteht darin, die freie Verbindung II in einem inerten, mit Wasser unmischbaren Lösemittel, z.B. einem Ether wie Diethylether, Diisopropylether, Methyl-tert.-butylether, einem Keton wie Aceton, einem Aromaten wie Toluol und den Xylolen, einem Halogenkohlenwasserstoff wie Methylenchlorid, Chloroform und 1,2-Dichlorethan, oder einem höheren Alkohol wie 2-Ethylhexanol, zu lösen und mit einem Metallhydrid, -hydroxid, -alkoxid oder -carbonat - fest oder in Wasser oder einem niederen Alkohol gelöst oder suspendiert - zu neutralisieren.

Das gebildete Cyclohexenonoximether-Metallsalz I scheidet sich dabei entweder fest aus der Reaktionsmischung aus oder wird nach Entfernen des Lösungsmittel - amorph oder kristallin - erhalten.

Eine weitere Variante besteht darin, II mit einer etwa äquimolaren Menge eines Alkoholats des gewünschten Metall-Ions umzusetzen, wobei dann der entsprechende Alkohol als Lösungsmittel dient. Das Alkoholat kann entweder direkt eingesetzt oder in situ aus Metall und Alkohol erzeugt werden.

Die Isolierung von I erfolgt in diesem Fall üblicherweise durch Einengen der Reaktionslösung oder durch Zugeben eines unpolaren Fällungsmittels, z.B. eines Aromaten wie Benzol, Toluol und den Xylolen, eines Kohlenwasserstoffes wie n-Pentan, n-Hexan, Petrolether und Cyclohexan oder einem Ether wie Diethylether, Diisopropylether und Methyl-tert.-butylether, und Abtrennen des kristallinen oder amorphen Wertprodukts.

Außerdem können aus Cyclohexenonoximether-Metallsalzen, die anstelle von R^{b} ein anderes Alkalimetallion aufweisen, durch Umsalzen andere Salze hergestellt werden. Hierzu versetzt man üblicherweise die wäßrige Lösung eines gut wasserlöslichen Salzes I, z.B. des Na-Salzes, mit einer (in der Regel wäßrigen) Lösung eines Metallhalogenids oder Metallsulfates. Gewünschtenfalls kann die Umsalzung in Gegenwart eines mit Wasser mischbaren Lösungsmittels, z.B. eines cyclischen Ethers wie Tetrahydrofuran und Dioxan oder eines niedrigen Alkohols, oder in Gegenwart eines mit Wasser nicht mischbaren Lösungsmittels, z.B. eines Aromaten wie Toluol und den Xylolen, eines Ethers wie Diethylether, Diisopropylether und Methyl-tert.-butylether, eines Ketons wie Aceton, eines halogenierten Kohlenwasserstoffs wie Methylenchlorid und Chloroform oder eines höheren Alkohols wie 2-Ethylhexanol, vorgenommen werden.

Sofern die Löslichkeit des gewünschten Salzes I in Wasser entsprechend gering ist, beginnt dabei bereits dessen Abscheidung aus dem Reaktionsgemisch.

Bei ausreichender Löslichkeit in einem organischen, mit Wasser nicht mischbaren Lösungsmittel, kann das gewünschte Salz I auch mit diesem Lösungsmittel extrahiert werden. Gelöstes Halogenid verbleibt dann üblicherweise in der wässrigen Phase.

Die Cyclohexenonoximether-Metallsalze I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die reinen Isomeren getrennt werden können. Reine optisch aktive Isomere lassen sich zweckmäßig aus entsprechenden optisch aktiven Ausgangsprodukten (II) herstellen.

Die Cyclohexenonoximether-Metallsalze I eignen sich - sowohl als Isomerengemische als auch in Form der reinen Isomeren - als Herbizide. Im allgemeinen sind sie verträglich und somit selektiv in breitblättrigen Kulturen sowie in monokotylen (einkeimblättrigen) Gewächsen, welche nicht zu den Gramineen zählen. Einige der erfindungsgemäßen Salze I sind auch zur selektiven Bekämpfung von unerwünschten Gräsern in Gramineenkulturen geeignet. Dieser Effekt tritt vor allem bei niedrigen Aufwandmengen auf.

In Abhängigkeit von der jeweiligen Applikationsmethode können die erfindungsgemäßen Mittel noch in einer weiteren Zahl von Kulturpflanzen zur Beseitigung unerwünschter Pflanzen eingesetzt werden. In Betracht kommen beispielsweise folgende Kulturen: Allium cepa, Ananas comosus, Arachis hypogaea, Asparagus officinalis, Beta vulgaris spec. altissima, Beta vulagris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Ficus elastica, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Cyclohexenonoximether-Metallsalze I auch in Kulturen, die durch Züchtung einschließlich gentechnischer Methoden gegen die Wirkung von Herbiziden tolerant sind, verwandt werden.

Die Cyclohexenonoximether-Metallsalze I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich dabei ganz nach den Verwendungszwecken; sie sollten in jedem Fall möglichst die feinste Verteilung der erfindungsgemäßen Salze I gewährleisten.

### Als inerte Hilfsstoffe kommen im wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden, etwa von 0,001 bis 98 Gew.%, vorzugsweise 0,01 bis 95 Gew.-%. Die Wirkstoffe werden dabei in einer Reinheit von 90% bis 100%, vorzugsweise 95% bis 100% (nach NMR-Spektrum) eingesetzt.

Die Applikation der erfindungsgemäßen Salze I bzw. deren Aufbereitung kann im Vorauflauf- oder im Nachauflaufverfahren erfolgen, vornehmlich durch Blattspritzung. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.). Dabei kann die Ausbringung z.B. mit Wasser als Trägerstoff durch übliche Spritztechniken mit Mengen von etwa 100 bis 1000 l/ha Spritzbrühe erfolgen. Eine Anwendung der herbiziden Mittel im sogenannten "Low Volume"- oder "Ultra-low-Volume"-Verfahren ist ebenso möglich wie ihre Applikation in Form von Granulaten.

Zur Verbreiterung des Wirkungsspektrums und zur Erzielung synergistischer Effekte können die Cyclohexenonoximether-Metallsalze I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Besonders geeignet sind N-(Butoxymethyl)-2-chlor-N-(2,6-diethylphenyl)-acetamid (Common name: Butachlor), 2-(1,3-Benzthiazol-2-yloxy)-N-methyl-acetanilid (Common name: Mefenacet), 3,7-Dichlorchinolin-8-carbonsäure (Common name: Quinclorac), $-(4,6-Dimethoxypyrimidin-2-yl-carbamoyl-sulfamoyl)-O-toluolsäure-methylester (Common name: Bensulfuronmethyl), 3-Isopropyl-lH-2,1,3-benzothiadiazin4-(3H)one-2,2-dioxide (Common name: Bentazon), N-(Ethylthio-carbonyl)-azepan (Common name: Molinate), N,N-Diethyl-carbaminsäure-4-chlorbenzylthioester (Common name: Thiobencarb), N-(2-Propoxyethyl)-2-chlor-N-(2,6-diethylphenyl)acetamid (Common name: Pretilachlor), 3,5-Bis(methylthio-carbonyl)-2-difluormethyl-4-(2-methylpropyl)-6-trifluormethyl-pyridin, (Common name: Dithiopyr), 2-[4-(6-Chlor-benzoxazol-2-yloxy)-phenoxy]-propionsäure-ethylester (Common name: Fenoxapropethyl), N-(2-Phenyl-prop-2-yl-thiocarbonyl)-piperidin (Common name: Dimepiperate), 4-(2,4-Dichlorbenzoyl)-1,3-dimethyl-pyrazol-5-yl-toluyl-4-sulfonat (Common name: Pyrazolynate, Pyrazolate), 2-[4-(2,4-Dichlorbenzoyl)-1,3-dimethyl-pyrazol-5-yloxy]-acetophenon (Common name: Pyrazoxyfen), 2-[4-(2,4-Dichlor-m-toluyl)-1,3-dimethylpyrazol-5-yloxy-4'-methylacetophenon (Common name: Benzofenap), 2-(2-Naphthyloxy)-propionanilid (Common name: Naproanilid), 5-(4,6-Dimethoxypyrimidin-2-yl-carbamoylsulfamoyl)-1-methylpyrazol-4-carbonsäuremethylester (Common name: Pyrazosulfuronethyl), 1-(4,6-Dimethoxy-1,3,5-triazin-2-yl)-3-[2-(2-methoxyethoxy) -phenylsulfonyl] -harnstoff (Common name: Cinosulfuron), 2-Brom-3,3-dimethyl-N-(1-methyl-l-phenylethyl)-butyramid (Common name: Bromobutide), 1-(1-methyl-l-phenylethyl)-3-p-toluyl-harnstoff (Common name: Dymron, Daimuron), N²-(1,2-Dimethylpropyl) -N⁴-ethyl-6-methylthio-1,3,5-triazin-2,4-diamin (Common name: Dimethametryn), S-Benzyl-1,2-dimethylpropyl(ethyl)-thiocarbamat (Common name: Esprocarb), (Z)-N-but-2-enyloxymethyl-2-chlor-2',6'-diethylacetanilid (Common name: Butenachlor), S-2-Methylpiperidinocarbonylmethyl-O,O-dipropyl-phosphorodithionat (Common name: Piperophos), (1RS,2SR,4SR)-1,4-Epoxy-p-menth-2-yl-2-methylbenzylether (Common name: Cinmethylin), N-(3,4-Dichlorphenyl)-propanamid (Common name: Propanil), a-Chlor-N-(3-methoxy-2-thienyl)-methyl-2',6'-dimethylacetanilid, 4-Ethoxybenz-2',3'-dihydrochloranilid, 1-Diethylcarbamoyl-3-(2,4,6-trimethylphenylsulfonyl)-1,2,4-triazol, 3-(2-Chlorphenylmethyl)-1(1-methyl-l-phenylethyl)-harnstoff, 2-(2-Chlor-4-mesylbenzoyl)-cyclohexan-1,3-dion, 2,4-Dichlorphenoxyessigsäure (Common name: 2,4-D), N-(2-Chlorimidazol[1,2-a]pyridin-3-yl-sulfonyl)-N'-(4,6-dimethoxy-2-pyrimidyl)harnstoff (Common name: Imazosulfuron), 1-{[2-(Cyclopropylcarbonyl)phenyl]aminosulfonyl}-3-(4,6-dimethoxypyrimidin-2-yl)harnstoff, 1-(4,6-Dimethoxypyrimidin-2-yl)-3-[1-methyl-4-(2-methyl-2H-tetrazol-5-yl)pyrazol-5-ylsulfonyl] harnstoff, 4-(4-Chlor-2-methylphenoxy)buttersäure (Common name: MCPB), 2,4-Bis(ethylamino)-6-methylthio-1,3,5-triazine (Common name: Simetryne), [[(4,6-dimethoxy-2-pyrimidinyl)amino]carbonyl]-2-ethoxyphenylester (Common name: Ethoxysulfuron).

Außerdem kann es von Nutzen sein, die Cyclohexenonoximether-Metallsalze I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### Herstellung von 2-{1-[2-(4-Chlorphenoxy)-propyloximino]-butyl}-5-tetrahydrothiopyran-3-yl-cyclohexan-1,3-dion-Lithiumsalz

Eine Lösung von 2-{1-[2-(4-Chlorphenoxy)-propyloximino]-butyl}-5-tetrahydrothiopyran-3-yl-cyclohexan-1,3-dion (300 g) in 900 ml Methyl-tert.-butylether wurde unter starkem Rühren zu einer Lösung von Lithiumhydroxid (16,8 g) in 500 ml Wasser gegeben. Danach wurde der entstandene Feststoffanteil abgetrennt und im Vakuumtrockenschrank getrocknet. Ausbeute: 285 g des Li-Salzes mit einer Reinheit von 97 %.

### Beispiel 2

### Herstellung von 2-{1-[2-(4-Chlorphenoxy)-propyloximino]-butyl}-5-tetrahydrothiopyran-3-yl-cyclohexan-1,3-dion-Lithiumsalz

Eine Lösung von Lithiumhydroxid (1,85 g) in 60 ml Wasser wurde unter starkem Rühren auf eine Lösung von 2-{1-[2-(4-Chlorphenoxy)-propyloximino]-butyl}-5-tetrahydrothiopyran-3-yl-cyclohexan-1,3-dion (43,5 g) in 100 ml Toluol gegeben, wonach man über Nacht bei Raumtemperatur rührte. Der entstandene Feststoffanteil wurde abgetrennt und im Vakuumtrockenschrank getrocknet.
Ausbeute: 28 g des Li-Salzes.

### Beispiel 3

### Herstellung von 2-{1-[2-(4-Chlorphenoxy)-propyloximino]-butyl}-5-tetrahydrothiopyran-3-yl-cyclohexan-1,3-dion-Lithiumsalz

Eine Lösung von Lithiumhydroxid (1,85 g) in 60 ml Wasser wurde unter starkem Rühren auf eine Lösung von 2-{1-[2-(4-Chlorphenoxy)-propyloximino]-butyl}-5-tetrahydrothiopyran-3-yl-cyclohexan-1,3-dion-Rohprodukt (56,7%ig; 43,5 g) in 100 ml Methyl-tert.-butylether gegeben. Nach 2 Std. Rühren bei Raumtemperaur wurde der entstandene Feststoffanteil abgetrennt und im Vakuumtrockenschrank getrocknet. Man erhielt 27,1 g des Li-Salzes mit einer Reinheit von 86,8 % (Aubeute: 94,3 % der Theorie).

### Beispiel 4

### Herstellung von 2-{1-[2-(4-Chlorphenoxy)-propyloximino]-butyl}-5-tetrahydrothiopyran-3-yl-cyclohexan-1,3-dion-Lithiumsalz durch Natrium/Lithium Austausch

Eine Lösung von Natriumhydroxid (2,64 g) in 90 ml Wasser wurde auf eine Lösung von 2-{1-[2-(4-Chlorphenoxy)-propyloximino]-butyl}-5-tetrahydrothiopyran-3-yl-cyclohexan-1,3-dion (90,5%ig; 32,4 g) in 100 ml Toluol gegeben. Nach ¼ Std. Rühren bei Raumtemperaur wurden die Phasen getrennt. Ausbeute: 142,4 g einer wäßrigen Lösung von 2-{1-[2-(4-Chlorphenoxy)-propyloximino]- butyl}-5-tetrahydrothiopyran-3-yl-cyclohexan-1,3-dion-Natriumsalz.

Die Hälfte der erhaltenen Natrium-Salzlösung wurde auf 10°C gekühlt und auf eine 10°C kalte Lösung von Lithiumchlorid (1,4 g) in 110 ml Wasser getropft. Dann rühte man über Nacht bei Raumtemperatur, wonach der Feststoffanteil abgetrennt und getrocknet wurde. Man erhielt 13,4 g des Li-Salzes mit einer Reinheit von 97,8% (Aubeute: 88% der Theorie).

Die zweite Hälfte der erhaltenen Natrium-Salzlösung wurde auf 10°C gekühlt und auf eine 10°C kalte Mischung aus 100 ml Methyl-tert.-butylether und einer Lösung von Lithiumchlorid (1,4 g) in 110 ml Wasser getropft. Anschließend rührte man das Gemisch über Nacht bei Raumtemperatur, wonach der Feststoffanteil abgetrennt und getrocknet wurde. Man erhielt 12,5 g des Li-Salzes mit einer Reinheit von 99,5% (Aubeute: 84% der Theorie).

### Beispiel 5 (Vergleich)

### Herstellung von 2-{1-[2-(4-Chlorphenoxy)-propyloximino]-butyl}-5-tetrahydrothiopyran-3-yl-cyclohexan-1,3-dion-Natriumsalz

Zu einer Lösung von 2-{1-[2-(4-Chlorphenoxy)-propyloximino]-butyl}-5-tetrahydrothiopyran-3-yl-cyclohexan-1,3-dion (12,4 g) in 10 ml Toluol wurde unter kräftigem Rühren eine Lösung von Natriumhydroxid (1,04 g) in 13,4 ml Wasser gegeben. Nach 20 Minuten trennte man die wässrige Phase ab und engte sie im Vakuum ein. Ausbeute: 11,5 g des gewünschten Natriumsalzes.

In einem weiteren Experiment wurde das Wertprodukt mittels Gefriertrocknung vom Wasser befreit.

### Beispiel 6 (Vergleich)

### Herstellung von 2-{1-[2-(4-Chlorphenoxy)-propyloximino]-butyl}-5-tetrahydrothiopyran-3-yl-cyclohexan-1,3-dion-Kaliumsalz

Beispiel 5 wurde unter Verwendung von 1,81 g Kaliumhydroxid anstelle von Natriumhydroxid wiederholt. Nach Einengen der wässrigen Phase im Vakuum erhielt man 12,0 g des gewünschten Kaliumsalzes.

### Beispiel 7 (Vergleich)

### Herstellung von 2-{1-[2-(4-Chlorphenoxy)-propyloximino]-butyl}-5-tetrahydrothiopyran-3-yl-cyclohexan-1,3-dion-Magnesiumsalz

Zu einer Lösung von 2-{1-[2-(4-Chlorphenoxy)-propyloximino]-butyl}-5-tetrahydrothiopyran-3-yl-cyclohexan-1,3-dion (698 g) in 1,5 1 Methanol tropfte man bei 20-30°C eine Lösung von Magnesiumethanolat (90 g) in 0,5 1 Methanol. Nach beendeter Zugabe wurde noch 2 Std. auf Rückflußtemperatur erhitzt. Dann destillierte man das Methanol ab. Restmengen an Methanol wurden durch Zugabe von Toluol und anschließendem Einengen der Mischung vollständig verdrängt. Zur Reinigung rührte man den Rückstand in Methyl-tert.-butylether auf und trennte ihn danach wieder ab. Nach Trocknung erhielt man 556 g des gewünschten Magnesiumsalzes.

Prüfung physikalischer Eigenschaften:

### 1) Bestimmung der Wasserdampfaufnahme (Hygroskopizität):

Zur Bestimmung der Wasserdampfaufnahme wurden die zu untersuchenden Wirkstoffproben zunächst 48 Std. lang bei 50°C im Vakuum getrocknet und anschließend bei einer Umgebungstemperatur von 20°C relativen Luftfeuchten von 32%, 52% oder 66% ausgesetzt. Über einen Zeitraum von 14 Tagen maß man dann die Gewichtszunahme der Wirkstoffproben.

Tabelle 2 zeigt die Wasserdampfaufnahme verschiedener Salze von 2-{1-[2-(4-Chlorphenoxy)-propyloximino]-butyl}-5-tetrahydrothiopyran-3-yl-cyclohexan-1,3-dion (prozentual zum Ausgangsgewicht):

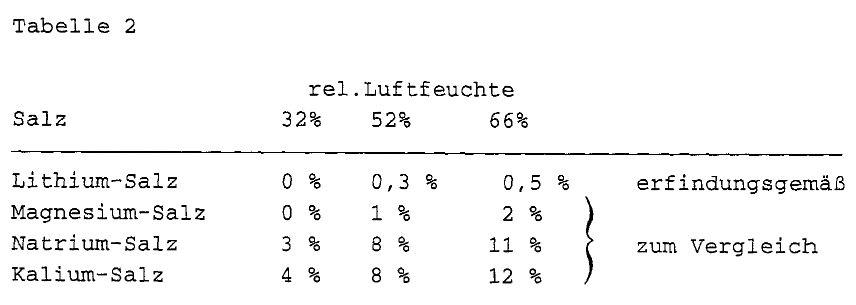

### 2) Bestimmung von DSC¹⁾-Kurven:

Gemessen wurde im Temperaturbereich von 20 bis 400°C mit einer Heizrate von 5 K/min. Vor der Messung wurden die Wirkstoffproben getrocknet. Zur Messung selbst wurde das Gerät DSC 200 der Fa. Netzsch verwendet.

Die DSC-Kurve des Lithiumsalzes von 2-{1-[2-(4-Chlorphenoxy)-propyloximino]-butyl}-5-tetrahydrothiopyran-3-yl-cyclohexan-1,3-dion zeigt eine Zersetzung erst oberhalb etwa 140°C.

### 3) Bestimmung der Lagerstabilität:

Hierzu wurden die verschiedenen Wirkstoffproben in fest verschlossen Glasgefäßen für eine bestimmte Zeit bei verschiedenen Temperaturen gelagert. Anschließend wurde der Wirktstoffgehalt der Proben bestimmt und mit dem Vergleichswert zu Beginn der Lagerung (Nullwert) verglichen. Tabelle 3 zeigt den Wirkstoffgehalt als relativen Anteil bezogen auf den Nullwert.

**Tabelle 3**

| Vergleich der Stabilität verschiedener Salze von 2-{1-[2-(4-Chlorphenoxy)-propyloximino]-butyl}-5-tetrahydrothiopyran-3-yl-cyclohexan-1,3-dion (II) nach 3 Monaten Lagerung: | | | | |
|---|---|---|---|---|
| | Lagerstabilität bei | | | |
| Salz | 20°C | 30°C | 40°C | 50°C |
| Lithium-Salz (erfindungsgemäß) | 100 % | 100 % | 100 % | 100 % |
| Natrium-Salz (Vergleich) | 100 % | 87 % | 43 % | -- |
| Magnesium-Salz (Vergleich) | 100 % | 98 % | 93 % | 81 % |
| 1) Differential Scanning Calorimetrie | | | | |

Bei der freien Verbindung (II) {als 87%iger techn. Wirkstoff} dagegen betrug der rel. Wirkstoffgehalt nach 3 Monaten Lagerung bei 20°C nur mehr 69 %.

### Formulierungsbeispiele:

### Beispiel 8: Emulsionskonzentrat

10 Gewichtsteile 2-{1-[2-(4-Chlorphenoxy)-propyloximino]-butyl}-5-tetrahydrothiopyran-3-yl-cyclohexan-1,3-dion-Lithiumsalz wurden in einer Mischung gelöst, die aus 80 Gewichtsteilen eines alkylierten Benzols und 20 Gewichtsteilen eines Anlagerungsproduktes von 8 mol Ethylenoxid an ein mol Nonylphenyl bestand. Man erhielt ein stabiles Emulsionskonzentrat.

### Beispiel 9: 5%ige Granulatformulierung

| | |
|---|---|
| 5 Gew.% | 2-{1-[2-(4-Chlorphenoxy)-propyloximino]-butyl}-5-tetrahydrothiopyran-3-yl-cyclohexan-1,3-dion-Magnesiumsalz, |
| 3 Gew.% | Phenolsulfonsäure-Formaldehyd-Kondensat, |
| 3 Gew.% | Phenol-Formaldehy-Sulfit-Kondensat, |
| 20 Gew.% | Natriummetasilikat und |
| add 100% | Kreide |

wurden innig vermischt mittels einer Rotor-Schnellmühle vermahlen. Anschließend wurde die Mischung mit Wasser angefeuchtet und mittels eines Korbextruders extrudiert. Das erhaltene Granulat wurde getrocknet. Nach Schnellagerung über 14 Tage bei 54°C betrug der Wirkstoffgehalt noch 88% vom Nullwert.

### Beispiel 10: Wasserdispergierbares Pulver

| | |
|---|---|
| 12,5 Gew.% | 2-{1-[2-(4-Chlorphenoxy)-propyloximino]-butyl}-5-tetrahydrothiopyran-3-yl-cyclohexan-1,3-dion-Lithiumsalz, |
| 7 Gew.% | Naphthalinsulfonsäure-Formaldehydkondensat, |
| 14 Gew.% | Natrium-Ligninsulfonat, |
| 3 Gew.% | Phenolsulfonsäure-Formaldehyd-Kondensat und |
| add 100% | Kreide |

wurden innig vermischt mittels einer Rotor-Schnellmühle vermahlen. Man erhielt ein wasserdispergierbares Pulver.

### Beispiel 11: Wasserdispergierbares Granulat

| | |
|---|---|
| 70 Gew.% | 2-{1-[2-(4-Chlorphenoxy)-propyloximino]-butyl}-5-tetrahydrothiopyran-3-yl-cyclohexan-1,3-dion-Lithiumsalz und |
| add 100 Gew.% | Naphthalinsulfonsäure-Formaldehydkondensat wurden innig vermischt und dann mittels einer Rotor-Schnellmühle vermahlen, angefeuchtet und mittels eines Korbextruders extrudiert. Das erhaltene Granulat wurde getrocknet. |

Nach Schnellagerung bei 54°C über 14 Tage betrug der Wirkstoffgehalt noch 99% des Ausgangswertes.

### Anwendungsbeispiel:

Die herbizide Wirkung der Cyclohexenonoximether-Metallsalze I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäß-en aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Echinochloa crus-galli | Hühnerhirse | barnyardgrass |
| Leptochloa filiformis | fadenförmiger Steifhalm | red sprangletop |
| Setaria viridis | Grüne Borstenhirse | green foxtail |

Bei einer Aufwandmenge von 0,25 kg/ha a.S. zeigte das 2-{1-[2-(4-Chlorphenoxy)-propyloximino]-butyl}-5-tetrahydrothiopyran-3-yl-cyclohexan-1,3-dion-Lithiumsalz im Nachauflaufverfahren eine sehr gute Wirkung gegen die o.g. Ungräser.

## Patentansprüche

1. Cyclohexenonoximether-Metallsalze der Formel I 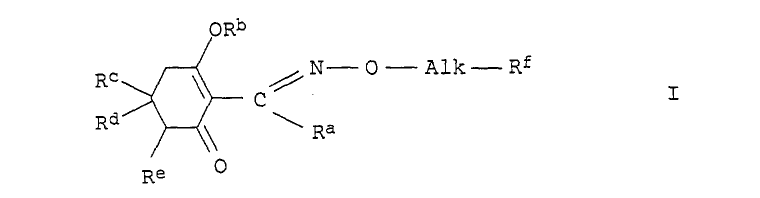 in der die Variablen folgende Bedeutungen haben:
R^{a} C₁-C₆-Alkyl;
R^{b} ein Lithiumion;
R^{c} einen 6- oder 7-gliedrigen gesättigten oder ein- oder zweifach ungesättigten Heterocyclus, der neben Kohlenstoffatomen ein oder zwei Sauerstoff- oder Schwefelatome oder ein Sauerstoff- und ein Schwefelatom als Ringglieder enthält, und der gewünschtenfalls noch einen bis drei Substituenten tragen kann, jeweils ausgewählt aus der Gruppe bestehend aus Hydroxyl, Halogen, C₁-C₄-Alkyl, C₁-C₄-Haloalkyl, C₁-C₄-Alkoxy und C₁-C₄-Alkylthio;
R^{d} Wasserstoff;
R^{e} Wasserstoff;
Alk eine 3- bis 6-gliedrige Alkylenkette, die gewünschtenfalls einen C₁-C₃-Alkylsubstituenten tragen kann und die neben Methylen- oder Methineinheiten ein Sauerstoff- oder Schwefelatom als Brückenglied enthält;
R^{f} Halogenphenyl.

2. Cyclohexenonoximether-Metallsalze der Formel I nach Anspruch 1, wobei
R^{a} für Ethyl oder n-Propyl und
R^{c} für Tetrahydropyran-3-yl, Tetrahydropyran-4-yl oder Tetrahydrothiopyran-3-yl
stehen.

3. Verwendung der Cyclohexenonoximether-Metallsalze der Formel I gemäß Anspruch 1 oder 2 als Herbizide.

4. Herbizides Mittel, enthaltend eine herbizid wirksame Menge mindestens eines Cyclohexenonoximether-Metallsalzes der Formel I gemäß Anspruch 1 oder 2 und mindestens einen inerten flüssigen und/oder festen Trägerstoff sowie gewünschtenfalls mindestens einen oberflächenaktiven Stoff.

5. Verfahren zur Herstellung von herbizid wirksamen Mitteln, **dadurch gekennzeichnet, daß** man eine herbizid wirksame Menge mindestens eines Cyclohexenonoximether-Metallsalzes der Formel I gemäß Anspruch 1 oder 2 und mindestens einen inerten flüssigen und/oder festen Trägerstoff sowie gewünschtenfalls mindestens einen oberflächenaktiven Stoff mischt.

6. Verfahren zur Bekämpfung von unerwünschtem Pflanzenwuchs, **dadurch gekennzeichnet, daß** man eine herbizid wirksame Menge mindestens eines Cyclohexenonoximether-Metallsalzes der Formel I gemäß Anspruch 1 oder 2 auf Pflanzen, deren Lebensraum oder auf Saatgut einwirken läßt.

7. Verfahren zur Herstellung von Cyclohexenonoximether-Metallsalzen der Formel I gemäß Anspruch 1, **dadurch gekennzeichnet, daß** man die entsprechenden freien Verbindungen II 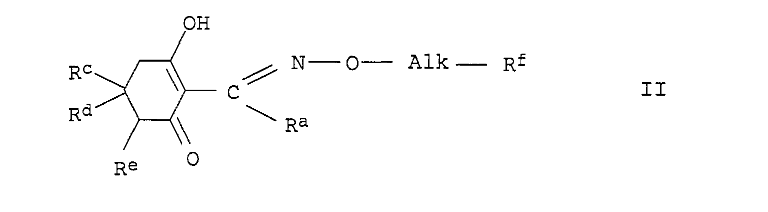 wobei R^{a}, R^{c} bis R^{f} und Alk die gleichen Bedeutungen wie bei Formel I haben, mit Lithiumhydroxid -hydrid, -(C₁-C₅-alkoxid) oder -carbonat umsetzt.

## Claims

1. A cyclohexenone oxime ether metal salt of the formula I 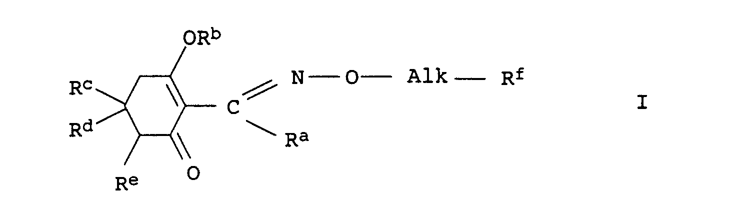 where the variables have the following meanings:
R^{a} is C₁-C₆-alkyl;
R^{b} is a lithium ion;
R^{c} is a 6- or 7-membered saturated or mono- or diunsaturated heterocycle which, in addition to carbon atoms, contains one or two oxygen or sulfur atoms or one oxygen and one sulfur atom as ring members and which, if desired, can additionally have attached to it one to three substituents, in each case selected from the group consisting of hydroxyl, halogen, C₁-C₄-alkyl, C₁-C₄-haloalkyl, C₁-C₄-alkoxy and C₁-C₄-alkylthio;
R^{d} is hydrogen;
R^{e} is hydrogen;
Alk is a 3- to 6-membered alkylene chain which, if desired, can have attached to it a C₁-C₃-alkyl substituent and which, in addition to methylene or methine units, contains an oxygen or sulfur atom as bridge member;
R^{f} is halophenyl.

2. A cyclohexenone oxime ether metal salt of the formula I as claimed in claim 1, where
R^{a} is ethyl or n-propyl, and
R^{c} is tetrahydropyran-3-yl, tetrahydropyran-4-yl or tetrahydrothiopyran-3-yl.

3. The use of the cyclohexenone oxime ether metal salt of the formula I as claimed in claim 1 or 2 as a herbicide.

4. A herbicidal composition comprising a herbicidally active amount of at least one cyclohexenone oxime ether metal salt of the formula I as claimed in claim 1 or 2 and at least one inert liquid and/or solid carrier and, if desired, at least one surfactant.

5. A process for the preparation of herbicidally active compositions, which comprises mixing a herbicidally active amount of at least one cyclohexenone oxime ether metal salt of the formula I as claimed in claim 1 or 2 and at least one inert liquid and/or solid carrier and, if desired, at least one surfactant.

6. A method of controlling undesirable vegetation, which comprises allowing a herbicidally active amount of at least one cyclohexenone oxime ether metal salt of the formula I as claimed in claim 1 or 2 to act on plants, their environment or on seed.

7. A process for the preparation of cyclohexenone oxime ether metal salts of the formula I as claimed in claim 1, which comprises reacting the corresponding free compounds II 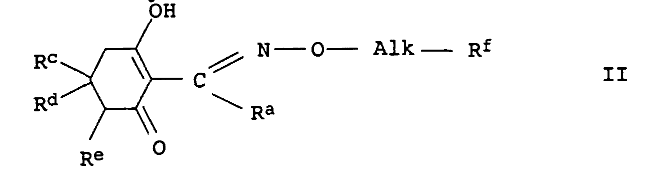 where R^{a}, R^{c} to R^{f} and alk have the same meanings as in formula I, with lithium hydroxide, lithium hydride, lithium (C₁-C₅-alkoxide) or lithium carbonate.

## Revendications

1. Sels métalliques d'éthers de cyclohexénonoximes de formule I 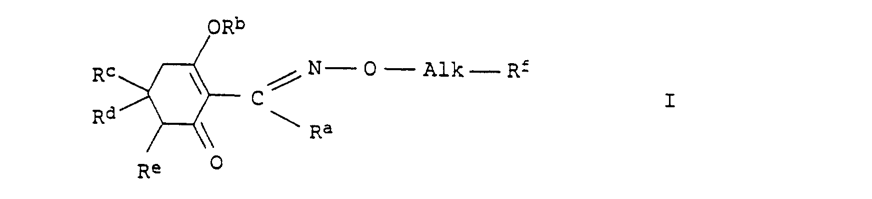 dans laquelle les symboles ont les significations suivantes :
R^{a} : un groupe alkyle en C1-C6 ;
R^{b} : un ion lithium ;
R^{c} : un hétérocycle à six ou sept chaînons, saturé, mono- ou di-insaturé qui, en plus des atomes de carbone, contient un ou deux atomes d'oxygène ou de soufre ou un atome d'oxygène et un atome de soufre en tant que chaînons cycliques et qui le cas échéant peut encore porter un à trois substituants choisis parmi les groupes hydroxy, les halogènes, les groupes alkyle en C1-C4, halogénoalkyle en C1-C4, alcoxy en C1-C4 et alkylthio en C1-C4 ;
R^{d} : l'hydrogène ;
R^{e} : l'hydrogène ;
Alk : une chaîne alkylène de trois à six chaînons qui peut le cas échéant porter un substituant alkyle en C1-C3 et, avec les motifs méthylène ou méthine, contient un pont consistant en un atome d'oxygène ou de soufre ;
R^{f} : un groupe halogénophényle.

2. Sels métalliques d'éthers de cyclohexénonoximes de formule I de la revendication 1 dans laquelle
R^{a} représente un groupe éthyle ou n-propyle et
R^{c} représente un groupe tétrahydropyran-3-yle, tétrahydropyran-4-yle ou tétrahydrothiopyran-3-yle.

3. Utilisation des sels métalliques d'éthers de cyclohexénonoximes de formule I de la revendication 1 ou 2 en tant qu'herbicides.

4. Produit herbicide contenant une quantité herbicide efficace d'au moins un sel métallique d'éther de cyclohexénonoxime de formule I de la revendication 1 ou 2 et au moins un véhicule inerte solide et/ou liquide avec le cas échéant au moins une substance tensio-active.

5. Procédé pour préparer des produits herbicides, **caractérisé par le fait que** l'on mélange une quantité herbicide efficace d'au moins un sel métallique d'éther de cyclohexénonoxime de formule I de la revendication 1 ou 2 et au moins un véhicule liquide et/ou solide inerte et, le cas échéant, au moins une substance tensio-active.

6. Procédé pour combattre les croissances de végétaux indésirables, **caractérisé par le fait que** l'on fait agir une quantité herbicide efficace d'au moins un sel métallique d'éther de cyclohexénonoxime de formule I de la revendication 1 ou 2 sur les végétaux, leur habitat ou leurs semences.

7. Procédé pour la préparation des sels métalliques d'éthers de cyclohexénonoximes de formule I de la revendication 1, **caractérisé par le fait que** l'on fait réagir les composés libres correspondants II 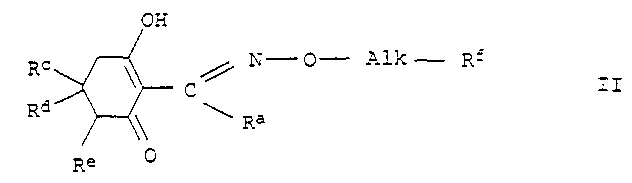 dans laquelle R^{a}, R^{c} à R^{f} et Alk ont les significations indiquées en référence à la formule I, avec l'hydroxyde de lithium, l'hydrure de lithium, un alcoolate en C1-C5 de lithium ou le carbonate de lithium.
